# EUROPEAN PATENT SPECIFICATION

(11) **EP 2 813 191 B1**
(45) Date of publication and mention of the grant of the patent: **23.08.2017**
(21) Application number: 13172767.9
(22) Date of filing: 19.06.2013
(51) Int. Cl.: A61B 17/42

(54) **Amnion insertion device**
Amnion-Einsatzvorrichtung
Dispositif d'insertion d'amnios

(30) Priority: 05.06.2013 KR 20130064669
(43) Date of publication of application: 17.12.2014
(73) Proprietor: Lee, Keun-Ho, Gyeonggi-do (KR); Lee, Keun-Young, Seocho-gu Seoul 06661 (KR)
(72) Inventor: Lee, Keun-Ho, Gyeonggi-do (KR); Lee, Keun-Young, Seocho-gu Seoul 06661 (KR)
(74) Representative: Gervasi, Gemma

(56) References cited:
- WO-A1-96/01604
- JP-A- H 114 835
- US-A- 6 126 675
- US-A1- 2002 183 777
- US-A1- 2012 130 394

## Description

### BACKGROUND OF THE INVENTION

### Field of the Invention

The present invention relates to an amnion insertion device, and more particularly to an amnion insertion device used in urgent cervical cerclage to treat cervical incompetence in which an expansion balloon is inflated by fluid pressurized by an air injection tube integrally combined with the inside of an external pipe and a through hole so as to push the bulging amnion into the uterine lumen without damage and then to stitch the cervix to complete treatment of cervical incompetence, the expansion balloon provided at the front portion of the air injection tube is not deformed and is not pushed backward during the process of pushing the bulging amnion into the uterine lumen so as to easily perform cervical cerclage, and the external pipe and the air injection tube are integrally provided so that an operator may operate the amnion insertion device while grasping the amnion insertion device with one hand and perform cervical cerclage with the other hand, thus maximizing efficiency of cervical cerclage.

### Description of the Related Art

As is well known, cervical incompetence is a medical condition in which the cervix is damaged and the amnion bulges without labor pains or bleeding during the second or third quarter of pregnancy due to a functional or structural cervical defect and consequently, pregnancy is ended as preterm delivery. Cervical incompetence occurs in 0.1-2% of pregnancies, is the cause of 15% of preterm deliveries between 16 and 28 weeks of pregnancy, and closely relates to morbidity and mortality of newborn infants.

As treatments for cervical incompetence, there are cervical cerclage, which is preventively used, and urgent cervical cerclage, which is used when the length of the cervix is excessively shortened, as determined through ultrasound diagnosis.

However, when cervical incompetence is not diagnosed and preventive cervical cerclage or urgent cervical cerclage is not carried out, if the cervix is damaged and the amnion bulges, delivery is inevitably performed.

As described above, if the cervix is damaged and the amnion bulges and protrudes to the outside of the cervix, urgent cervical cerclage is the best treatment option. Urgent cervical cerclage is an operation method in which the protruding amnion is pushed into the uterine lumen and then the cervix is stitched.

In order to push the amnion protruding to the outside of the cervix into the uterine lumen, a method using the Trendelenburg position, a method of injecting a saline solution into the bladder, and a method of pushing the amnion into the uterine lumen using sponge forceps or a Foley catheter are used.

Here, in case of the method using the Trendelenburg position, a pregnant woman lies on her back under the condition that her feet are raised so that the amnion may be naturally inserted into the uterine lumen. In case of the method of injecting a saline solution into the bladder, a designated amount of the saline solution is injected into the bladder so that the amnion is pushed upward by the saline solution. In case of the method using sponge forceps, wet gauze is picked up with the sponge forceps and then the amnion is inserted into the uterine lumen using the sponge forceps.

From among these conventional methods of cervical cerclage, the method using sponge forceps is mainly used. However, the structurally sharp gauze may damage the amnion and particularly, when the wet gauze is taken out of the uterine lumen after the amnion is inserted into the uterine lumen, the amnion may protrude again from the uterine lumen.

Further, the conventional method using a Foley catheter may be used if the cervix is slightly opened. However, if the amnion bulges, the Foley catheter may warp, and thus it may be difficult to push the amnion into the uterine lumen using the Foley catheter.

Document US2012/130394 discloses an amnion insertion device according to the preamble of claim 1. Therefore, in order to solve the above problems, Korean Patent No. 710905 discloses a conventional amnion insertion device. The conventional amnion insertion device disclosed in Korean Patent No. 710905, as exemplarily shown in FIG. 1, includes an air injection tube 10 into which air is injected by the action of an air injection unit 12 formed at one end of the air injection tube 10, an expansion balloon 30 formed at the other end of the air injection tube 10 and inflated by air injected through the air injection tube 10 to directly push the protruding amnion into the uterine lumen, a guide pipe 40 into which the air injection tube 10 except for the part of the air injection tube 10 provided with the expansion balloon 30 is inserted and which prevents warpage of the air injection tube 10 so as to easily push the amnion into the uterine lumen, and an air hole 50 formed at one side of the air injection tube 10 provided with the expansion balloon 30 to discharge air injected through the air injection tube 10 to the expansion balloon 30. Particularly, such a conventional amnion insertion device is characterized in that the expansion balloon 30 is fixed to the guide pipe 40 using an adhesive in order to prevent the inflated expansion balloon 30 from moving leftward and rightward by force pushing the protruding amnion into the uterine lumen.

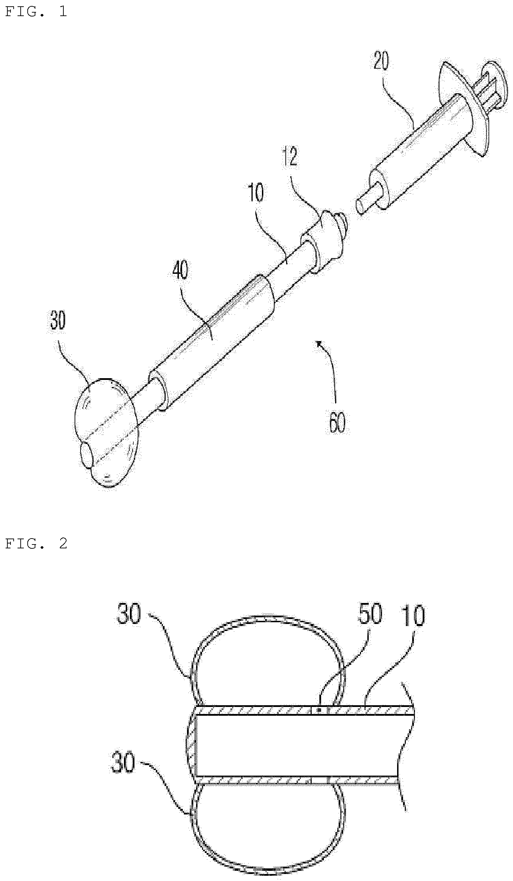

However, the conventional amnion insertion device disclosed in Korean Patent No. 710905 is configured such that the expansion balloon 30 is attached to the outer diameter of the front end of the air injection tube 10, as exemplarily shown in FIG. 2, and thus, even if a part of the rear portion of the expansion balloon 30 is fixed to the guide pipe 40, the expansion balloon 30 inevitably moves leftward and rightward or is pushed backward during the process of pushing the bulging amnion into the uterine lumen, and the operating process of pushing the bulging amnion into the uterine lumen may be difficult.

Particularly, in the conventional amnion insertion device disclosed in Korean Patent No. 710905, since the air injection tube 10 serving as a passage to inject air into the expansion balloon 30 and the guide pipe 40 combined with the outer circumferential surface of the air injection tube 10 are not integrated, instead being separately provided, one person pushes the bulging amnion into the uterine lumen by operating the amnion insertion device while taking the guide pipe 40 of the amnion insertion device with one hand and gripping the air injection tube 10 with the other hand during operation of cervical cerclage. That is, if cervical cerclage is carried out using the amnion insertion device disclosed in Korean Patent No. 710905, a person operating cervical cerclage and a person operating the amnion insertion device are respectively required, increasing labor costs, and when the two technicians do not operate in harmony, unexpected operation results may occur.

Further, in the amnion insertion device disclosed in Korean Patent No. 710905, the expansion balloon 30 is very thin and may be pushed backward or move leftward and rightward during the operation process of pushing the bulging amnion into the uterine lumen, and thus inflation of the expansion balloon 30 is limited. Therefore, if a patient who has an excessively protruding amnion and thus is damaged when the amnion is excessively pushed into the uterine lumen is operated, amniocentesis in which amniotic fluid is partially removed from the amnion to prevent damage to the amnion is first carried out so as to reduce the size of the amnion and then the protruding amnion is pushed into the uterine lumen using the amnion insertion device.

Moreover, in the conventional amnion insertion device, when the amnion is pushed into the uterine lumen, the expansion balloon 30 may not easily close the uterine lumen due to non-uniformity and elasticity of the entometrium and thus the amnion may protrude to the outside through such a gap. Also, the content of documents US 2002 183777, WO 96 01604, and US 6 126 675 is known. In particular, document US 2012 130394 shows an amnion insertion device comprising the technical features from the preamble of claim 1.

### SUMMARY OF THE INVENTION

Therefore, the present invention has been made in view of the above problems, and it is an object of the present invention to provide an amnion insertion device used in urgent cervical cerclage to treat cervical incompetence in which the cervix is damaged and the amnion bulges during pregnancy, in which an expansion balloon is inflated by fluid pressurized by an air injection tube inserted into an external pipe and a through hole so as to push the bulging amnion into the uterine lumen without damage and then to stitch the cervix to complete treatment of cervical incompetence, the expansion balloon provided at the front portion of the air injection tube is not deformed in terms of shape and is not pushed backward during the process of pushing the bulging amnion into the uterine lumen so as to easily perform cervical cerclage, and the external pipe and the air injection tube are integrally provided so that an operator may operate the amnion insertion device while grasping the amnion insertion device with one hand and perform cervical cerclage with the other hand, thus maximizing efficiency of cervical cerclage.

It is another object of the present invention to provide an amnion insertion device used in cervical cerclage, which properly adjusts the inflation degree of an expansion balloon according to the protruding degree of the amnion without amniocentesis in which amniotic fluid is partially removed from the amnion even if the amnion excessively protrudes.

It is yet another object of the present invention to provide an amnion insertion device which allows an operator to easily adjust the inflation degree of an expansion balloon with one hand.

In accordance with the present invention, the above and other objects can be accomplished by the provision of an amnion insertion device (100) including an air injection tube (130) into which fluid is injected by the action of an air injection part (120) provided at one end of the air injection tube (130), an expansion balloon (160) formed at the other end of the air injection tube (130), inflated by the fluid injected through the air injection tube (130) and directly pushing the protruding amnion into the uterine lumen, an external pipe (140) into which the air injection tube (130) except for the part of the air injection tube 130 provided with the expansion balloon (160) is integrally inserted, and a supporter (150) provided between the external pipe (140) and the expansion balloon (160).

A designated air injection unit (102) may be connected to one end of the air injection tube (130), and a designated amount of the fluid injected by the operation of the air injection unit (102) may pass through a through hole (132) formed at a front end (134) of the air injection tube (130) and be injected into the spherical expansion balloon (160) so as to inflate the expansion balloon (160).

The expansion balloon (160) may be configured such that a first bonding part (168) is formed under the condition that a part of the concave (166) is inserted into the front end (134) of the air injection tube (130), and a second binding part (152) is formed by inserting a part of the end of a rear inclined part (164) of the expansion balloon (160) into a front end (154) of the supporter (150) formed of one selected from the group consisting of latex, PVC, silicon, and polyurethane.

The air injection tube (130) may be integrally inserted into the external pipe (140) serving as a handle so that the protruding amnion may be easily pushed into the uterine lumen using the expansion balloon (160) inflated by the air injection part (120), and be formed of one selected from the group consisting of latex, latex coated with silicon, PVC, silicon, and polyurethane.

The expansion balloon (160) may be formed of one selected from the group consisting of latex, latex coated with silicon, PVC, polyurethane, and silicon, and be inflated into a spherical shape provided with a concave (166) formed at the front portion of the expansion balloon (160) by the fluid injected through the air injection tube (130).

Scales to measure the insertion depth of the amnion may be displayed on the surface of the external pipe (140), the front end of the external pipe (140) may be bent outward to form an inclined part (142) such that the outer circumferential surface of the inclined part (142) is combined with the rear portion of the supporter (150) connected to the expansion balloon (160), and the external pipe (140) may have a diameter preventing the air injection tube (130) inserted into the external pipe (140) from moving and be formed of one selected from the group consisting of FRP, PVC, metal, wood, and plastic

A syringe-type air injector (110) or an air injection unit (200) including an air pump (202) and performing electromotive air injection and discharge may be combined with the rear end of the air injection part (120).

The air injection unit (200) may include the air pump (202) provided with a fan motor formed therein, rotated in regular and reverse directions, and performing air injection and discharge, a connection hose (204) connected to one side surface of the air pump (202) and transmitting air injected and discharged through the air pump (202), and an operating unit (206) connected to one end of the connection hose (204) and provided with an operating switch (210) formed at one side thereof to execute air injection and discharge.

The operating switch (210) may be slidable on the operating unit (206), and an injection operating region (214) may be formed at the front end of the operating unit (206), a discharge operating region (216) may be formed at the rear end of the operating unit (206), and a stopping region (212) may be formed between the injection operating region (214) and the discharge operating region (216) so that an air injection or discharge signal may be generated according to the position of the operating switch (210).

An injection speed indicator (218) on which scales indicating injection speed are displayed may be formed in the horizontal direction below the injection operating region (214) and the discharge operating region (216) on the operating unit (206).

### BRIEF DESCRIPTION OF THE DRAWINGS

The above and other objects, features and other advantages of the present invention will be more clearly understood from the following detailed description taken in conjunction with the accompanying drawings, in which:
FIG. 1 is a perspective view of a conventional amnion insertion device;
FIG. 2 is a sectional view of a state in which an expansion balloon of the conventional amnion insertion device is inflated;
FIG. 3 is a perspective view of an amnion insertion device in accordance with one embodiment of the present invention;
FIG. 4a is a sectional view illustrating a state before an expansion balloon of the amnion insertion device in accordance with the embodiment of the present invention is inflated;
FIG. 4b is a sectional view illustrating a state in which the expansion balloon of the amnion insertion device in accordance with the embodiment of the present invention is inflated;
FIG. 5 is a perspective view illustrating application of another air injection unit to the amnion insertion device in accordance with the embodiment of the present invention; and
FIG. 6 is a view illustrating the configuration of an operating unit of the air injection unit of FIG. 5.

### DETAILED DESCRIPTION OF THE INVENTION

Now, preferred embodiments in accordance with the present invention will be described in detail with reference to the annexed drawings.

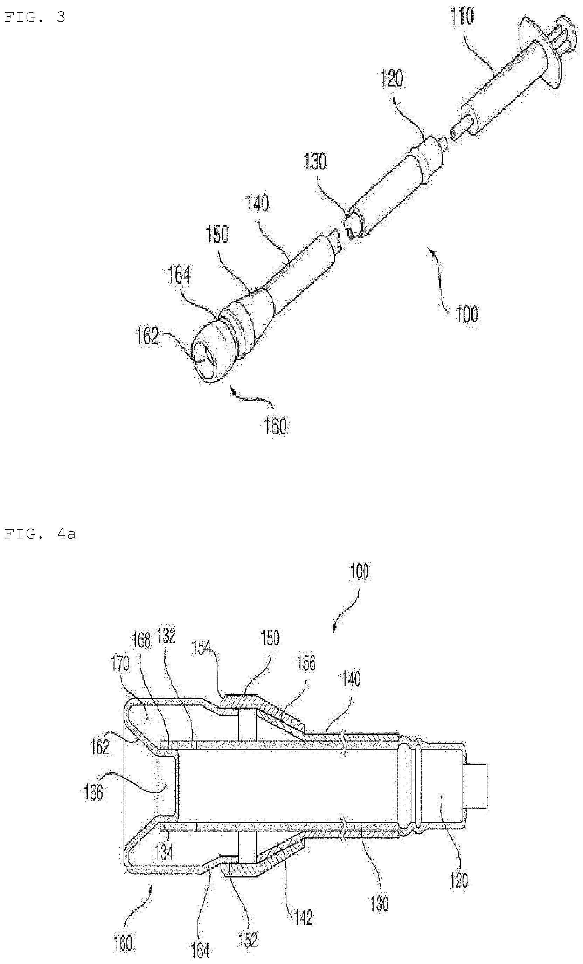

FIG. 3 is a perspective view of an amnion insertion device in accordance with one embodiment of the present invention.

With reference to FIG. 3, the amnion insertion device in accordance with the embodiment of the present invention is used in urgent cervical cerclage to treat cervical incompetence in which the cervix is damaged and the amnion bulges during pregnancy, and is configured such that an expansion balloon inflated by fluid pressurized by an air injection tube inserted into an external pipe and a through hole is formed in a spherical shape provided with a concave formed at the front portion thereof, thus being used in cervical cerclage in which the bulging amnion is pushed into the uterine lumen without damage and the cervix is stitched to treat cervical incompetence.

Cervical incompetence occurs due to congenital factors, damage to the cervix, hormone deficiency, bacterial infections, etc.

As treatments for such cervical incompetence, there are cervical cerclage which is preventively used and urgent cervical cerclage which is used when the length of the cervix is excessively shortened, as determined through ultrasound diagnosis.

In case of cervical incompetence in which the cervix is damaged and the amnion bulges and protrudes to the outside of the cervix, as described above, cervical cerclage in which the protruding amnion is pushed into the uterine lumen and then the cervix is stitched is carried out as the best treatment.

The amnion insertion device in accordance with the embodiment of the present invention which is used in cervical cerclage, includes an air injection tube into which air is injected by the action of an air injection unit provided at one end of the air injection tube, an expansion balloon formed at the other end of the air injection tube and inflated by the fluid injected through the air injection tube, an external pipe into which the air injection tube except for the part of the air injection tube provided with the expansion balloon is integrally inserted, and a supporter provided between the external pipe and the expansion balloon.

Here, the expansion balloon is formed in a spherical shape provided with the concave formed at the front portion thereof, and directly contacts the amnion to push the protruding amnion into the uterine lumen.

The external pipe serves as a handle preventing warpage of the air injection tube integrally combined with the inside of the external pipe to facilitate pushing of the amnion into the uterine lumen, when the protruding amnion is pushed into the uterine lumen using the inflated expansion balloon, and may be formed any solid material, such as fiberglass reinforced plastic (FRP), PVC, metal, wood, or plastic, and be particularly formed of plastic.

Further, although not shown in the drawings, scales to measure the insertion depth of the amnion are displayed on the surface of the external pipe.

Hereinafter, one embodiment of the present invention will be described in detail with reference to the annexed drawings.

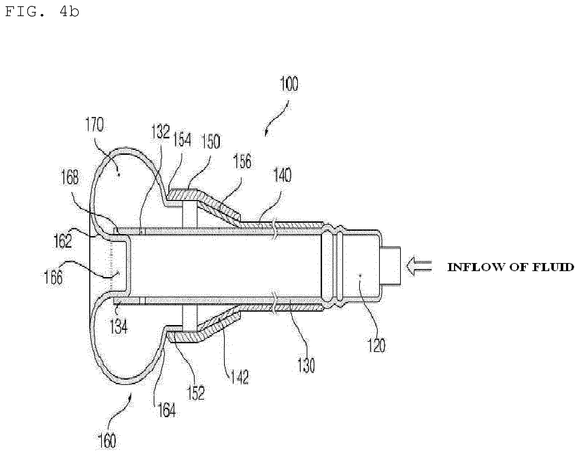

FIG. 3 is a perspective view of an amnion insertion device in accordance with one embodiment of the present invention, FIG. 4a is a sectional view illustrating a state before an expansion balloon of the amnion insertion device in accordance with the embodiment of the present invention is inflated, and FIG. 4b is a sectional view illustrating a state in which the expansion balloon of the amnion insertion device in accordance with the embodiment of the present invention is inflated.

An amnion insertion device 100 in accordance with one embodiment of the present invention which is used in cervical cerclage, as exemplarily shown in FIG. 3, includes an air injection tube 130 into which fluid is injected by the action of an air injection part 120 provided at one end of the air injection tube 130, an expansion balloon 160 formed at the other end of the air injection tube 130, inflated by the fluid injected through the air injection tube 130 and directly pushing the protruding amnion into the uterine lumen, an external pipe 140 into which the air injection tube 130 except for the part of the air injection tube 130 provided with the expansion balloon 160 is integrally inserted, and a supporter 150 provided between the external pipe 140 and the expansion balloon 160.

A designated air injection unit 102 is connected to one end of the air injection tube 130, and a designated amount of fluid injected by the operation of the air injection unit 102 passes through a through hole 132 and is injected into the spherical expansion balloon 160 provided with a concave 166 formed on the front portion thereof so as to inflate the expansion balloon 160. The expansion balloon 160 is configured such that a first bonding part 168 is formed under the condition that a part of the concave 166 is inserted into a front end 134 of the air injection tube 130, and a second binding part 152 is formed by inserting a part of the end of a rear inclined part 164 of the expansion balloon 160 into a front end 154 of the supporter 150 formed of one selected from the group consisting of latex, PVC, silicon, and polyurethane.

Further, the air injection tube 130 is integrally inserted into the external pipe 140 serving as a handle so that the protruding amnion may be easily pushed into the uterine lumen using the expansion balloon 160 inflated by the air injection part 120. The diameter of the air injection tube 130 may be about 4∼8mm but is not limited thereto. Further, the air injection tube 130 may be formed of one selected from the group consisting of latex, latex coated with silicon, PVC, silicon, and polyurethane.

The air injection part 120 is inserted into one end of the air injection tube 130, and serves to inject fluid, such as air, into the air injection tube 130 using an air injector 110, such as a syringe, provided at the outside to induce inflation of the expansion balloon 160.

With reference to FIG. 4a, the expansion balloon 160 is provided at the outside of the front end of the air injection tube 130, and may be formed of one selected from the group consisting of latex, latex coated with silicon, polyurethane, silicon, and PVC. The fluid of a designated amount injected into an expansion part 170 formed at an arbitrary position of the air injection tube 130 inflates the expansion balloon 160.

The expansion balloon 160 inflated by the fluid injected through the air injection tube 130 may be formed in a spherical shape or oval shape having the concave 166 formed at the front portion of the expansion balloon 160. When the expansion balloon 160 is formed in a spherical shape having the concave 166 formed at the front portion of the expansion balloon 160, the area of a portion of the expansion balloon 160 contacting the amnion increases, and thus, the amnion may be more stably pushed into the uterine lumen.

The external pipe 140 serves as a handle preventing warpage of the air injection tube 130 inserted into the external pipe 140 and facilitating pushing of the amnion into the uterine lumen, when the protruding amnion caused by cervical incompetence is pushed into the uterine lumen using the expansion balloon 160 inflated by the fluid injected through the air injection tube 130.

Such an external pipe 140 may have a diameter preventing the air injection tube 130 inserted into the external pipe 140 from moving, and be formed of any solid material, such as FRP, PVC, metal, wood, or plastic, and be particularly formed of plastic.

Conventionally, when the expansion balloon 160 is inflated to push the protruding amnion into the uterine lumen, the inflated expansion balloon 160 moves leftward and rightward by pushing force. In order to prevent such a problem, the amnion insertion device 100 in accordance with the present invention is configured such that the front portion of the supporter 150 having a diameter greater than the diameter of the external pipe 140 stably supports the circumference of the rear portion of the expansion balloon 160 and has a great thickness so as to prevent sagging of the expansion balloon 160 to more stably push the amnion into the uterine lumen without damage to the amnion.

The through hole 132, as shown in FIG. 4a, is formed around the front end 134 of the air injection tube 130 with which the concave 166 of the expansion balloon 160 is combined, and thus, the fluid supplied through the air injection tube 130 may inflate the expansion balloon 160 via the through hole 132.

Hereinafter, the operation of the above-described amnion insertion device used in cervical cerclage in accordance with the present invention will be described.

If cervical incompetence, in which the cervix is damaged and the amnion bulges and protrudes to the outside of the cervix due to congenital factors, damage to the cervix, hormone deficiency, bacterial infections, etc. occurs during pregnancy, cervical cerclage, in which the protruding amnion is directly pushed into the uterine lumen or a designated amount of amniotic fluid is removed from the amnion to reduce the size of the amnion and then the protruding amnion is pushed into the uterine lumen, and then the cervix is stitched, is carried out to treat cervical incompetence.

When such cervical cerclage is carried out, the protruding amnion is pushed into the uterine lumen using the amnion insertion device in accordance with the present invention. The operation of the amnion insertion device in accordance with the present invention is as follows.

First, when air is injected into the air injection tube 130 through the air injection part 120 using the air injector 110, such as a syringe, the fluid injected through the through hole 132 formed around the front end 134 of the air injection tube 130 is injected into the expansion part 170 of the expansion balloon 160, and the expansion balloon 160 in accordance with the present invention is inflated into a spherical shape such that the concave 166 formed at the front portion of the expansion balloon 160 is maintained and the rear portion of the expansion balloon 160 is gradually inflated outward, as exemplarily shown in FIG. 4b.

When the supporter 150 is pushed forward under the condition that a front inclined part 162 of the expansion balloon 160 inflated into the spherical shape having the concave 166 formed at the front portion thereof directly contacts the protruding amnion, the protruding amnion may be stably pushed into the uterine lumen without damage to the amnion by force of the expansion balloon 160 provided at the front portion of the air injection tube 130 integrally formed within the supporter 150 such that the rear portion of the expansion balloon 160 is supported by the supporter 150 so as not to be pushed backward. In such an amnion insertion device 100 in accordance with the present invention, the air injection tube 130 inflating the expansion balloon 160 and the external pipe 140 including the supporter 150 stably supporting the rear portion of the expansion balloon 160 are integrally combined, and thus, an operator may perform all operations of the amnion insertion device 100 in accordance with the present invention with one hand.

The conventional amnion insertion device may damage the amnion, when the excessively protruding amnion is pushed into the uterine lumen. Therefore, in order to prevent such a problem, amniocentesis in which amniotic fluid is partially removed from the amnion is carried out so as to reduce the size of the amnion, and then the protruding amnion is pushed into the uterine lumen using the amnion insertion device. On the other hand, in case of the amnion insertion device 100 in accordance with the present invention, the expansion balloon 160 has a great thickness and is configured such that the front portion of the expansion balloon 160 forms the concave 166 and the rear portion of the expansion balloon 160 is stably supported by the supporter 150, and thus, the expansion balloon 160 is not pushed backward or does not move leftward and rightward during the process of pushing the amnion into the uterine lumen and the inflation degree of the expansion balloon 160 may be properly adjusted according to the protruding degree of the amnion. Consequently, if the amnion insertion device 100 in accordance with the present invention is used, any separate operation to adjust the size of the amnion is not required during cervical cerclage.

Thereafter, when the amnion is completely inserted into the uterine lumen, the operator completes cervical cerclage by stitching the cervix by the other hand. Thus, the operator may effectively perform cervical cerclage.

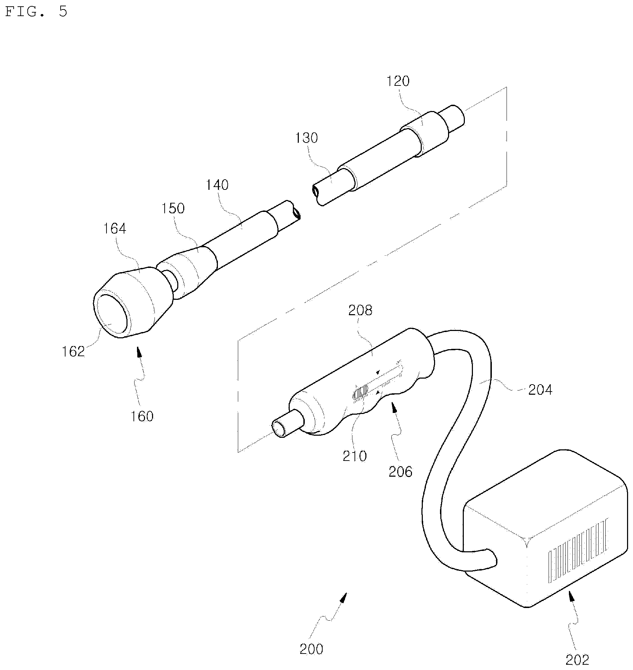

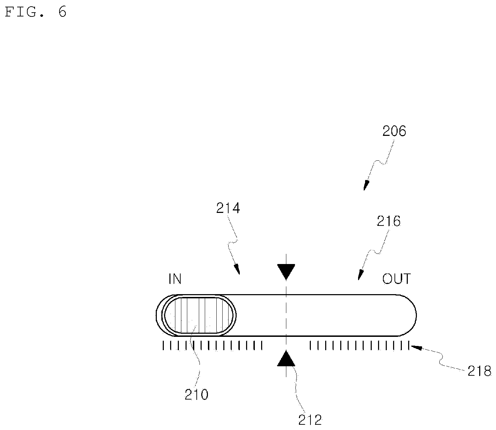

FIG. 5 is a perspective view illustrating application of another air injection unit to the amnion insertion device in accordance with the embodiment of the present invention, and FIG. 6 is a view illustrating the configuration of an operating unit of the air injection unit of FIG. 5.

With reference to FIGs. 5 and 6, the amnion insertion device 100 in accordance with the embodiment of the present invention may use an air injection unit 200 allowing an operator to easily adjust the inflation degree of the expansion balloon 160 with one hand, as a substitute for the air injector 110.

That is, the amnion insertion device 100 in accordance with the embodiment of the present invention includes the air injection part 120 to inject air into the expansion balloon 160, and one of the syringe-type air injector 110 and the air injection unit 200 including an air pump 202 and performing electromotive air injection and discharge may be combined with the rear end of the air injection part 120.

Since the syringe-type air injector 110 has been described above, the air injection unit 200 including an electric device will be described below with reference to FIGs. 5 and 6.

The air injection unit 200 includes the air pump 202 provided with a fan motor (not shown) formed therein, rotated in regular and reverse directions and performing air injection and discharge, a connection hose 204 connected to one side surface of the air pump 202 and transmitting air injected and discharged through the air pump 202, and an operating unit 206 connected to one end of the connection hose 204 and provided with an operating switch 210 formed at one side thereof to execute air injection and discharge.

The fan motor (not shown) is a well known motor which is rotatable in regular and reverse directions and provided with a variable resistor (not shown) or an inverter (not shown) formed therein, and the air pump 202 provided with the fan motor (not shown) formed therein is also well known.

The operating switch 210 is slidable on the operating unit 206, and an injection operating region 214 is formed at the front end of the operating unit 206, a discharge operating region 216 is formed at the rear end of the operating unit 206, and a stopping region 212 is formed between the injection operating region 214 and the discharge operating region 216 so that an air injection or discharge signal may be generated according to the position of the operating switch 210.

That is, in a state in which the air pump 202 is powered on, the air pump 202 is not operated when the operating switch 210 is located in the stopping region 212, the air pump 202 is rotated in a regular direction to inject air into the expansion balloon 160 when the operating switch 210 is located in the injection operating region 214, and the air pump 202 is rotated in the reverse direction to discharge air from the expansion balloon 160 to the outside when the operating switch 210 is located in the discharge operating region 216.

Further, an injection speed indicator 218 on which scales indicating injection speed are displayed is formed in the horizontal direction below the injection operating region 214 and the discharge operating region 216 on the operating unit 206.

Therefore, since an operator may properly adjust the inflation degree of the expansion balloon according to the protruding degree of the amnion by operating the operating switch 210 while grasping the amnion insertion device with one hand, any separate operation to adjust the size of the amnion during cervical cerclage is not required. Further, one operator may easily adjust the inflation degree of the expansion balloon with one hand, thus easily executing cervical cerclage.

As apparent from the above description, one embodiment of one embodiment of the present invention provides an amnion insertion device in which the entirety of the rear portion of an expansion balloon provided at the front portion of an air injection tube is stably supported by a supporter so as to prevent the expansion balloon in an inflated state from being deformed or pushed backward during a process of pushing the bulging amnion into the uterine lumen and to easily perform cervical cerclage. Further, since an external pipe and the air injection tube are formed integrally, and thus an operator may individually operate the external pipe and the air injection tube while grasping the amnion insertion device with one hand and perform cervical cerclage with the other hand so as to maximize efficiency of cervical cerclage.

Further, the amnion insertion device in accordance with the embodiment of the present invention is configured such that the expansion balloon has a great thickness and is formed of an elastic material, the front portion of the expansion balloon forms a concave, and the rear portion of the expansion balloon is stably supported by the supporter, and may properly adjust the inflation degree of the expansion balloon according to the protruding degree of the amnion without amniocentesis in which amniotic fluid is partially removed from the amnion even if the amnion excessively protrudes, thus not requiring any separate operation to adjust the size of the amnion during cervical cerclage.

Further, the amnion insertion device in accordance with the embodiment of the present invention allows one operator to easily adjust the inflation degree of the expansion balloon with one hand, thus allowing the operator to easily perform cervical cerclage.

Although the preferred embodiments of the present invention have been disclosed for illustrative purposes, those skilled in the art will appreciate that various modifications, additions and substitutions are possible, without departing from the scope of the invention as disclosed in the accompanying claims.

## Claims

1. An amnion insertion device (100) comprising:
an air injection tube (130) into which fluid is injected by the action of an air injection part (120) provided at one end of the air injection tube (130);
an expansion balloon (160) formed at the other end of the air injection tube (130), inflated by the fluid injected through the air injection tube (130), and directly pushing a protruding amnion into the uterine lumen;
an external pipe (140) into which the air injection tube (130) except for the part of the air injection tube (130) provided with the expansion balloon (160) is integrally inserted; and
a supporter (150) provided between the external pipe (140) and the expansion balloon (160);
**characterized in that** it comprises an air injection unit (200) comprising an air pump (202) and configured to perform electromotive air injection and discharge which is combined with the rear end of the air injection part (120);
wherein the air injection unit (200) includes the air pump (202) provided with a fan motor formed therein, rotated in regular and reverse directions, and performing air injection and discharge, a connection hose (204) connected to one side surface of the air pump (202) and transmitting air injected and discharged through the air pump (202), and an operating unit (206) connected to one end of the connection hose (204) and provided with an operating switch (210) formed at one side thereof to execute air injection and discharge;
wherein the operating switch (210) is slidable on the operating unit (206), and an injection operating region (214) is formed at the front end of the operating unit (206), a discharge operating region (216) is formed at the rear end of the operating unit (206), and a stopping region (212) is formed between the injection operating region (214) and the discharge operating region (216) so that an air injection or discharge signal may be generated according to the position of the operating switch (210).

2. The amnion insertion device (100) according to claim 1, wherein the expansion balloon is spherical; a designated air injection unit (200) being connected to one end of the air injection tube (130) via the connection with the rear end of the air injection part (120), and a designated amount of the fluid injected by the operation of the air injection unit (102) passes through a through hole (132) formed at a front end (134) of the air injection tube (130) and is injected into the expansion balloon (160) so as to inflate the expansion balloon (160).

3. The amnion insertion device (100) according to claim 1, wherein scales to measure the insertion depth of the amnion are displayed on the surface of the external pipe (140), the front end of the external pipe (140) is bent outward to form an inclined part (142) such that the outer circumferential surface of the inclined part (142) is combined with the rear portion of the supporter (150) connected to the expansion balloon (160), and the external pipe (140) has a diameter preventing the air injection tube (130) inserted into the external pipe (140) from moving and is formed of one selected from the group consisting of FRP, PVC, metal, wood, and plastic.

4. The amnion insertion device (100) according to claim 1, wherein an injection speed indicator (218) on which scales indicating injection speed are displayed is formed in the horizontal direction below the injection operating region (214) and the discharge operating region (216) on the operating unit (206).

## Patentansprüche

1. Amnioneinsetzvorrichtung (100), umfassend:
ein Luftinjektionsrohr (130), in das Fluid durch die Wirkung eines Luftinjektionsteiles (120), das an einem Ende des Luftinjektionsrohres (130) vorgesehen ist, injiziert wird;
einen Ausdehnungsballon (160), der an dem anderen Ende des Luftinjektionsrohres (130) gebildet, durch das Fluid aufgeblasen wird, das durch das Luftinjektionsrohr (130) injiziert wird, und ein vorragendes Amnion direkt in das Uterusvolumen drückt;
ein äußeres Rohr (140), in das das Luftinjektionsrohr (130) mit der Ausnahme desjenigen Teils des Luftinjektionsrohres (130), das mit dem Ausdehnungsballon (160) versehen ist, integral eingesetzt ist; und
einen Träger (150), der zwischen dem Außenrohr (140) und dem Ausdehnungsballon (160) vorgesehen ist;
**dadurch gekennzeichnet, dass** sie eine Luftinjektionseinheit (200) umfasst, die eine Luftpumpe (202) umfasst und derart konfiguriert ist, eine elektromotorische Luftinjektion/Austragung auszuführen, die mit dem rückwärtigen Ende des Luftinjektionsteils (120) kombiniert ist;
wobei die Luftinjektionseinheit (200) umfasst: die Luftpumpe (202), die mit einem darin gebildeten Gebläsemotor versehen ist, der in regulären und Rückwärtsrichtungen drehbar ist und eine Luftinjektion und -austragung ausführt, einen Verbindungsschlauch (204), der mit einer Seitenfläche der Luftpumpe (202) verbunden ist und durch die Luftpumpe (202) injizierte und ausgetragene Luft überträgt, und eine Betriebseinheit (206), die mit einem Ende des Verbindungsschlauchs (204) verbunden ist und mit einem Betriebsschalter (210) versehen ist, der an einer Seite davon gebildet ist, um eine Luftinjektion und -austragung auszuführen;
wobei der Betriebsschalter (210) an der Betriebseinheit (206) verschiebbar ist, und ein Injektionsbetriebsgebiet (214) an dem vorderen Ende der Betriebseinheit (206) gebildet ist, ein Austragsbetriebsgebiet (216) an dem rückwärtigen Ende der Betriebseinheit (206) gebildet ist, und ein Stoppgebiet (212) zwischen dem Injektionsbetriebsgebiet (214) und dem Austragsbetriebsgebiet (216) gebildet ist, so dass ein Luftinjektions- oder Austragssignal gemäß der Position des Betriebsschalters (210) erzeugt werden kann.

2. Amnioneinsetzvorrichtung (100) nach Anspruch 1, wobei der Ausdehnungsballon kugelförmig ist; eine bereitgestellte Luftinjektionseinheit (200) mit einem Ende des Luftinjektionsrohres (130) über die Verbindung mit dem rückwärtigen Ende des Luftinjektionsteils (120) verbunden ist, und eine festgelegte Menge des Fluides, die durch den Betrieb der Luftinjektionseinheit (102) injiziert wird, durch ein Durchgangsloch (132) gelangt, das an einem vorderen Ende (134) des Luftinjektionsrohres (130) gebildet ist, und in den Ausdehnungsballon (160) injiziert wird, um so den Ausdehnungsballon (160) aufzublasen.

3. Amnioneinsetzvorrichtung (100) nach Anspruch 1, wobei Skalen, um die Einsetztiefe des Amnions zu messen, an der Fläche des Außenrohres (140) angezeigt sind, das vordere Ende des Außenrohres (140) nach außen gebogen ist, um ein schräggestelltes Teil (142) zu bilden, so dass die äußere Umfangsfläche des schräggestellten Teils (142) mit dem rückwärtigen Abschnitt des Trägers (150) kombiniert ist, der mit dem Ausdehnungsballon (160) verbunden ist, und das Außenrohr (140) einen Durchmesser aufweist, der verhindert, dass das Luftinjektionsrohr (130), das in das Außenrohr (140) eingesetzt ist, sich bewegt, und aus einem Material gebildet ist, das aus der Gruppe gewählt ist, die FKV, PVC, Metall, Holz und Kunststoff umfasst.

4. Amnioneinsetzvorrichtung (100) nach Anspruch 1, wobei eine Injektionsgeschwindigkeitsanzeigeeinrichtung (218), an der Skalen, die eine Injektionsgeschwindigkeit angeben, angezeigt sind, in der horizontalen Richtung unterhalb des Injektionsbetriebsgebiets (214) und des Austragsbetriebsgebietes (216) an der Betriebseinheit (206) gebildet ist.

## Revendications

1. Dispositif d'insertion d'amnios (100) comprenant :
un tube d'injection d'air (130) dans lequel un fluide est injecté par l'action d'une partie d'injection d'air (120) disposée à une extrémité du tube d'injection d'air (130) ;
un ballonnet de dilatation (160) formé à l'autre extrémité du tube d'injection d'air (130), gonflé par le fluide injecté par l'intermédiaire du tube d'injection d'air (130), et poussant directement un amnios saillant dans la lumière utérine ;
un tuyau externe (140) dans lequel le tube d'injection d'air (130) à l'exception de la partie du tube d'injection d'air (130) pourvue du ballonnet de dilatation (160) est intégralement inséré ; et
un élément de support (150) disposé entre le tuyau externe (140) et le ballonnet de dilatation (160) ; **caractérisé en ce qu'**il comprend une unité d'injection d'air (200) comprenant une pompe à air (202) et configurée pour effectuer une injection et une décharge d'air électromotrice qui est combinée avec l'extrémité arrière de la partie d'injection d'air (120) ;
dans lequel l'unité d'injection d'air (200) comprend la pompe à air (202) pourvue d'un moteur de ventilateur formé dans celle-ci, mise en rotation dans des directions normale et inverse, et effectuant une injection et une décharge d'air, un tuyau flexible de raccordement (204) raccordé à une surface latérale de la pompe à air (202) et transmettant l'air injecté et déchargé par l'intermédiaire de la pompe à air (202), et une unité opérationnelle (206) raccordée à une extrémité du tuyau flexible de raccordement (204) et pourvue d'un commutateur de fonctionnement (210) formé à un côté de celle-ci pour effectuer l'injection et la décharge d'air ;
dans lequel le commutateur de fonctionnement (210) peut coulisser sur l'unité de fonctionnement (206), et une région d'opération d'injection (214) est formée à l'extrémité avant de l'unité de fonctionnement (206), une région d'opération de décharge (216) est formée à l'extrémité arrière de l'unité de fonctionnement (206), et une région d'arrêt (212) est formée entre la région d'opérations d'injection (214) est la région d'opération de décharge (216) de sorte qu'un signal d'injection de décharge d'air puisse être généré en fonction de la position du commutateur de fonctionnement (210).

2. Dispositif d'insertion d'amnios (100) selon la revendication 1, dans lequel le ballonnet de dilatation est sphérique ; une unité d'injection d'air désignée (200) étant raccordée à une extrémité du tube d'injection d'air (130) par l'intermédiaire du raccordement avec l'extrémité arrière de la partie d'injection d'air (120), et une quantité désignée du fluide injectée par le fonctionnement de l'unité d'injection d'air (102) traverse un trou traversant (132) formé à une extrémité avant (134) du tube d'injection d'air (130) et est injectée dans le ballonnet de dilatation (160) de façon à gonfler le ballonnet de dilatation (160).

3. Dispositif d'insertion d'amnios (100) selon la revendication 1, dans lequel des graduations pour mesurer la profondeur d'insertion de l'amnios sont présentées sur la surface du tuyau externe (140), l'extrémité avant du tuyau externe (140) est fléchie vers l'extérieur pour former une partie inclinée (142) de sorte que la surface circonférentielle externe de la partie inclinée (142) soit combinée avec la partie arrière de l'élément de support (150) raccordée au ballonnet de dilatation (160), et le tuyau externe (140) a un diamètre empêchant le tube d'injection d'air (130) inséré dans le tuyau externe (140) de se déplacer et est formé de l'un choisi dans le groupe constitué de FRP, PVC, métal, bois et matière plastique.

4. Dispositif d'insertion d'amnios (100) selon la revendication 1, dans lequel un indicateur de vitesse d'injection (218) sur lequel des graduations indiquant la vitesse d'injection sont présentées est formé dans la direction horizontale au-dessous de la région d'opération d'injection (214) et la région d'opération de décharge (216) sur l'unité de fonctionnement (206).
